(19) Europäisches Patentamt
European Patent Office
Office européen des brevets

(11) **EP 4 345 127 A1**

(12) **EUROPEAN PATENT APPLICATION**
published in accordance with Art. 153(4) EPC

(43) Date of publication:
**03.04.2024 Bulletin 2024/14**

(21) Application number: **23788596.7**

(22) Date of filing: **12.04.2023**

(51) International Patent Classification (IPC):
*C08J 11/18* (2006.01)    *C08J 11/28* (2006.01)
*C08J 11/16* (2006.01)    *C07C 37/00* (2006.01)
*C07C 39/16* (2006.01)    *C07D 317/38* (2006.01)

(52) Cooperative Patent Classification (CPC):
**C07C 37/00; C07C 39/16; C07D 317/38;
C08J 11/16; C08J 11/18; C08J 11/28;** Y02W 30/62

(86) International application number:
**PCT/KR2023/004933**

(87) International publication number:
**WO 2023/200247 (19.10.2023 Gazette 2023/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.04.2022  KR 20220045959
04.04.2023  KR 20230044308**

(71) Applicant: **LG Chem, Ltd.
Seoul 07336 (KR)**

(72) Inventors:
• **LEE, Hyunyoung**
  **Daejeon 34122 (KR)**
• **PARK, Jun Beom**
  **Daejeon 34122 (KR)**
• **KIM, Hyun Cheol**
  **Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **MONOMER COMPOSITION FOR SYNTHESIZING RECYCLED PLASTIC, METHOD FOR MANUFACTURING SAME, AND RECYCLED PLASTIC AND MOLDED PRODUCT USING SAME**

(57)    The present invention relates to a monomer composition for synthesizing recycled plastic which comprises an aromatic diol compound, wherein a ratio of aromatic diol compound derivative impurity according to Equation 1 is 10% or less, the monomer composition has a color coordinate b* of 0.01 to 2, and the monomer composition is recovered from a polycarbonate-based resin, a preparation method thereof, and a recycled plastic, and molded product using the same.

EP 4 345 127 A1

**Description**

**[TECHNICAL FIELD]**

CROSS-REFERENCE TO RELATED APPLICATION(S)

This application claims the benefit of Korean Patent Application No. 10-2022-0045959 filed on April 13, 2022 and Korean Patent Application No. 10-2023-0044308 filed on April 4, 2023 in the Korean Intellectual Property Office, the entire contents of which are incorporated herein by reference.

The present invention relates to a monomer composition for synthesizing recycled plastic that contains an aromatic diol compound having high purity and excellent optical properties recovered through recycling by chemical decomposition of a polycarbonate-based resin, a preparation method thereof, and a recycled plastic and molded product using the same.

**[BACKGROUND ART]**

Polycarbonate is a thermoplastic polymer and is a plastic having excellent characteristics such as excellent transparency, ductility, and relatively low manufacturing cost.

Although polycarbonate is widely used for various applications, environmental and health concerns during waste treatment have been continuously raised.

Currently, a physical recycling method is carried out, but in this case, a problem accompanying the deterioration of the quality occurs, and thus, research on the chemical recycling of polycarbonate is underway.

Chemical decomposition of polycarbonate refers to obtaining an aromatic diol compound (e.g., bisphenol A; BPA) as a monomer through decomposition of polycarbonate, and then utilizing it again in polymerization to obtain a high-purity polycarbonate.

For such a chemical decomposition, thermal decomposition, hydrolysis, and alcohol decomposition are typically known. Among these, the most common method is alcohol decomposition using a base catalyst, but in the case of methanol decomposition, there is a problem that methanol harmful to the human body is used, and in the case of ethanol, there is a problem that high temperature and high pressure conditions are required and the yield is not high.

**[DETAILED DESCRIPTION OF THE INVENTION]**

**[Technical Problem]**

It is an object of the present invention to provide a monomer composition for synthesizing recycled plastic that can secure an aromatic diol compound having high purity and excellent optical properties recovered through recycling by chemical decomposition of a polycarbonate-based resin.

It is another object of the present invention to provide a method for preparing the monomer composition for synthesizing recycled plastic, a recycled plastic, and molded product using the monomer composition for synthesizing recycled plastic.

**[Technical Solution]**

In order to achieve the above object, provided herein is a monomer composition for synthesizing recycled plastic, which comprises an aromatic diol compound, wherein a ratio of aromatic diol compound derivative impurity according to the following Equation 1 is 10% or less, wherein the monomer composition has a color coordinate b* of 0.01 to 2, and wherein the monomer composition is recovered from a polycarbonate-based resin.

[Equation 1]

Ratio of aromatic diol compound derivative impurity (%) = (Peak area of the aromatic diol compound derivative in HPLC / Total peak area in HPLC) X 100.

Also provided herein is a method for preparing the monomer composition for synthesizing recycled plastic, the method comprising the steps of: adding polycarbonate to an organic solvent to prepare a mixed solution; adding a glycol-based compound and an inorganic base catalyst to the mixed solution and stirring them; and obtaining an aromatic diol compound formed in the stirring step.

Further provided herein is a recycled plastic comprising a reaction product of the monomer composition for synthesizing recycled plastic and a comonomer.

Further provided herein is a molded product comprising the recycled plastic.

Below, a monomer composition for synthesizing recycled plastic, a preparation method thereof, a recycled plastic, and molded product using the same according to specific embodiments of the present invention will be described in more detail.

Unless explicitly stated herein, the technical terms used herein are for the purpose of describing specific embodiments only and is not intended to limit the scope of the invention.

Singular expressions used herein may include the plural expressions unless they are differently expressed contextually.

It should be understood that the terms "comprise," "include", "have", etc. are used herein to specify the presence of stated feature, region, integer, step, action, element and/or component, but do not preclude the presence or addition of one or more other feature, region, integer, step, action, element, component and/or group.

Further, the terms including ordinal numbers such as "a first", "a second", etc. are used only for the purpose of distinguishing one component from another component, and are not limited by the ordinal numbers. For instance, a first component may be referred to as a second component, or similarly, the second component may be referred to as the first component, without departing from the scope of the present invention.

As used herein, the term "substituted or unsubstituted" means being unsubstituted or substituted with one or more substituent groups selected from the group consisting of deuterium; a halogen group; a cyano group; a nitro group; a hydroxy group; a carbonyl group; an ester group; an imide group; an amino group; a primary amino group; a carboxy group; a sulfonic acid group; a sulfonamide group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkoxysilylalkyl group; an arylphosphine group; and a heterocyclic group containing at least one of N, O and S atoms, or being unsubstituted or substituted with a substituent group to which two or more substituent groups of the above-exemplified substituent groups are linked. For example, "a substituent group in which two or more substituents are linked" may be a biphenylyl group. Namely, a biphenylyl group may be an aryl group, or it may be interpreted as a substituent group in which two phenyl groups are linked.

As used herein, the alkyl group is a monovalent functional group derived from an alkane, and may be a straight-chain or a branched-chain. The number of carbon atoms of the straight chain alkyl group is not particularly limited, but is preferably 1 to 20. Also, the number of carbon atoms of the branched chain alkyl group is 3 to 20. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, 2,6-dimethylheptane-4-yl and the like, but are not limited thereto. The alkyl group may be substituted or unsubstituted, and when substituted, examples of the substituent are the same as described above.

As used herein, the alkylene group is a bivalent functional group derived from alkane, and the aforementioned description of the alkyl group can be applied except that these are divalent functional groups. For example, the linear or branched alkylene group may include methylene group, ethylene group, propylene group, isobutylene group, sec-butylene group, tert-butylene group, pentylene group, hexylene group, and the like. The alkylene group can be substituted or unsubstituted.

As used herein, the fused ring means a cyclic structure in which mutually adjacent rings share only two atoms which form each ring, in a polycyclic system composed of two or more carbocyclic or heterocyclic rings.

As used herein, the "one or more" means, for example, "1, 2, 3, 4, or 5, particularly 1, 2, 3, or 4, more particularly 1, 2, or 3, and more particularly 1 or 2".

## 1. Monomer Composition for Synthesizing Recycled Plastic

According to one embodiment of the present invention, there can be provided a monomer composition for synthesizing recycled plastic, which comprises an aromatic diol compound, wherein a ratio of aromatic diol compound derivative impurity according to the following Equation 1 is 10% or less, wherein the monomer composition has a color coordinate b* of 0.01 to 2, and wherein the monomer composition is recovered from a polycarbonate-based resin.

[Equation 1]

Ratio of aromatic diol compound derivative impurity (%) = (Peak area of the aromatic diol compound derivative in HPLC / Total peak area in HPLC) X 100.

The present inventors have found through experiments that although the monomer composition for synthesizing recycled plastics of the one embodiment was recovered through recycling by chemical decomposition of the polycarbonate-based resin, the composition satisfies excellent optical properties together with the characteristics that it has high purity and significantly reduced impurity at the newly synthesized aromatic diol compound level, thereby capable of realizing excellent physical properties in the synthesis of polycarbonate-based resins using the same, and completed the present invention.

The present invention can have technical features that a composition comprising an aromatic diol compound can be obtained with high purity through recycling by chemical decomposition of polycarbonate-based resin.

Specifically, the monomer composition for synthesizing recycled plastics of the one embodiment is characterized by being recovered from a polycarbonate-based resin. That is, this means that recovery is performed from the polycarbonate-based resin in order to obtain the monomer composition for synthesizing recycled plastics of the one embodiment, and as a result, the monomer composition for synthesizing recycled plastics containing the aromatic diol compound is obtained together.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to thereby contain two or more carbonates, a copolymer can be synthesized. The homopolymer or copolymer can include all of low-molecular compounds, oligomers, and polymers depending on the molecular weight range.

Further, the monomer composition for synthesizing recycled plastics of the one embodiment may include an aromatic diol compound. Specific examples of the aromatic diol compound include bis(4-hydroxyphenyl)methane, bis(4-hydroxyphenyl)ether, bis(4-hydroxyphenyl)sulfone, bis(4-hydroxyphenyl)sulfoxide, bis(4-hydroxyphenyl)sulfide, bis(4-hydroxyphenyl)ketone, 1,1-bis(4-hydroxyphenyl)ethane, 2,2-bis(4-hydroxyphenyl)propane (bisphenol A), 2,2-bis(4-hydroxyphenyl)butane, 1,1-bis(4-hydroxyphenyl)cyclohexane (bisphenol Z), 2,2-bis(4-hydroxy-3,5-dibromophenyl)propane, 2,2-bis(4-hydroxy-3,5-dichlorophenyl)propane, 2,2-bis(4-hydroxy-3-bromophenyl)propane, 2,2-bis(4-hydroxy-3-chlorophenyl)propane, 2,2-bis(4-hydroxy-3-methylphenyl)propane, 2,2-bis(4-hydroxy-3,5-dimethylphenyl)propane, 1,1-bis(4-hydroxyphenyl)-1-phenylethane, or a mixture of two or more thereof, and the like. Preferably, the aromatic diol compound of the monomer composition for synthesizing recycled plastics of the one embodiment may be 2,2-bis(4-hydroxyphenyl)propane (bisphenol A).

The aromatic diol compound is characterized by being recovered from the polycarbonate-based resin used for recovering the monomer composition for synthesizing the recycled plastic. That is, this means that recovery is performed from the polycarbonate-based resin in order to obtain the monomer composition for synthesizing recycled plastics of the one embodiment, and as a result, an aromatic diol compound is also obtained together. Therefore, apart from the recovery from the polycarbonate-based resin in order to prepare the monomer composition for synthesizing recycled plastics of the one embodiment, the case where a novel aromatic diol compound is added from the outside is not included in the category of aromatic diol compound of the present invention.

Specifically, "being recovered from the polycarbonate-based resin" means being obtained through a depolymerization reaction of the polycarbonate-based resin. The depolymerization reaction can be carried out under acidic, neutral or basic conditions, and particularly, the depolymerization reaction can proceed under basic (alkaline) conditions. Particularly, the depolymerization reaction can be preferably carried out in the presence of a glycol-based compound, as will be described later.

The derivative of the aromatic diol compound may comprise one or more compounds selected from the group consisting of monohydroxyethyl-bisphenol A and bishydroxyethyl-bisphenol A.

Specifically, the monomer composition for synthesizing recycled plastic may have the ratio of the aromatic diol compound derivative impurity according to Equation 1 whose upper limit range is 10% or less, or 5% or less, or 1% or less, or 0.5% or less, or 0.43% or less, and whose lower limit range is 0.01% or more, or 0.05% or more, or 0.1% or more, or 0.15% or more, or 0.2% or more, or 0.25% or more, or 0.3% or more, or 0.35% or more, or 0.40% or more. A numerical range from the lower limit to the upper limit can also be satisfied by combining the upper limit range and the lower limit range. In one example of the numerical range of the lower limit to the upper limit, the ratio of the aromatic diol compound derivative impurity according to Formula 1 may be 0.01% to 10%.

According to Equation 1, %, which is a unit of the ratio of the aromatic diol compound derivative impurity, is a peak area ratio in HPLC, and means area%.

In Equation 1, the peak area of the aromatic diol compound derivative in HPLC is the total peak area of each of one or more aromatic diol compound derivatives. For example, when the aromatic diol compound derivative is a mixture of two types of monohydroxyethyl-bisphenol A and bishydroxyethyl-bisphenol A, it means the total peak area of each of monohydroxyethyl-bisphenol A and bishydroxyethyl-bisphenol A.

More specifically, the monohydroxyethyl-bisphenol A [MHE-BPA] may exhibit a peak at HPLC retention time of 2.84 minutes, and the bishydroxyethyl-bisphenol A [BHE-BPA] may exhibit a peak at an HPLC retention time of 2.61 minutes.

Examples of the method for measuring the weight ratio of derivative impurity of the aromatic diol compound of the monomer composition for synthesizing recycled plastics of the one embodiment is not particularly limited, and for example, high performance liquid chromatography (HPLC) analysis can be used. As for the specific methods, conditions, equipment, and the like of the HPLC, various well-known contents can be applied without limitation. However, as an example, the recycled bisphenol A monomer composition was dissolved at 1w% in acetonitrile (ACN) solvent under the conditions of normal pressure and 20~30°C, and then the weight ratio can be measured via a Waters HPLC system (e2695 separation module, 2998 PDA detector) using a UG120 (4.6 mm I.DX 50 mm). More specifically, the weight ratio can be measured under the conditions of ① Column: UG 120 (4.6mm I.DX 50mm), ② Column Temp: 40°C, (3) Injection volume: 10μℓ, ④ Flow: THF:ACN:water=15:15:70, volume=1.23ml/min (total=10min), and (5) Detector: 245nm.

In this manner, in the monomer composition for synthesizing recycled plastics of the one embodiment, the content of derivative impurities of aromatic diol compounds other than aromatic diol compounds, which are the main object materials to be recovered, is remarkably reduced, thereby capable of achieving excellent physical properties when synthesizing a polycarbonate-based resin using the same.

Meanwhile, the monomer composition for synthesizing recycled plastics of the one embodiment may have a color coordinate b* value of 0.01 to 2, or 0.5 to 1.2, or 0.58 to 1.02. That is, the monomer composition for synthesizing recycled plastic of the one embodiment may have a color coordinate b* value whose lower limit range is 0.01 or more, or 0.5 or more, or 0.58 or more, or 0.6 or more, or 0.7 or more, or 0.8 or more, or 0.9 or more, or 1.0 or more, and whose upper limit range is 2 or less, or 1.2 or less, or 1.02 or less. A numerical range from the lower limit to the upper limit can also be satisfied by combining the upper limit range and the lower limit range.

Further, the monomer composition for synthesizing recycled plastics of the one embodiment may have a color coordinate L* value of 94 to 99, or 95 to 98, or 96 to 98, or 96.554 to 97.207. That is, the monomer composition for synthesizing recycled plastic of the one embodiment may have a color coordinate L* value whose lower limit range is 94 or more, or 95 or more, or 96 or more, or 96.3 or more, or 96.554 or more, or 96.6 or more, or 96.9 or more, or 97.2 or more, and whose upper limit range is 99 or less, or 98 or less, or 97.207 or less. A numerical range from the lower limit to the upper limit can also be satisfied by combining the upper limit range and the lower limit range.

Further, the monomer composition for synthesizing recycled plastics of the one embodiment may have a color coordinate a* value of 0.01 to 1.5, or 0.1 to 1, or 0.10 to 0.23. That is, the monomer composition for synthesizing recycled plastic of the one embodiment may have a color coordinate a* value whose lower limit value range is 0.01 or more, or 0.1 or more, or 0.12 or more, or 0.14 or more, or 0.16 or more, or 0.18 or more, or 0.2 or more, or 0.22 or more, and whose upper limit range is 1.5 or less, or 1 or less, or 0.23 or less. A numerical range from the lower limit to the upper limit can also be satisfied by combining the upper limit range and the lower limit range.

As used herein, the "color coordinates" means coordinates in the CIE Lab color space, which are color values defined by CIE (Commossion International de l'Eclairage), and an arbitrary position in the CIE color space may be represented by three coordinate values, i.e., L*, a*, and b*.

Here, the L* value represents brightness, when L*=0, it represents black, and when L*=100, it represents white. In addition, the a* value represents a color having a corresponding color coordinate that leans toward one of pure red and pure green, and the b* value represents a color having a corresponding color coordinate that leans toward one of pure yellow and pure blue.

Specifically, the a* value is in the range of -a to +a. A maximum value of a* (a* max) represents pure red, and a minimum value of a* (a* min) represents pure green. Further, the b* value is in the range of -b to +b. A maximum value of b* (b* max) represents pure yellow, and a minimum value of b* (b* min) represents pure blue. For example, a negative b* value represents a color leaning toward pure blue, and a positive b* value represents color leaning toward pure yellow. When comparing b*=50 with b*=80, b*=80 is closer to pure yellow than b*=50.

When the color coordinate b* value of the monomer composition for synthesizing recycled plastics of the one embodiment excessively increases to more than 2, the monomer composition for synthesizing recycled plastics of the one embodiment represents a color excessively leaning toward yellow, resulting in deterioration of the color characteristics. Further, when the color coordinate b* value of the monomer composition for synthesizing recycled plastics of the one embodiment excessively decreases to less than 0.01, the monomer composition for synthesizing recycled plastics of the one embodiment represents a color excessively leaning toward blue, resulting in deterioration of the color characteristics.

When the color coordinate L* value of the monomer composition for synthesizing recycled plastics of the one embodiment excessively decreases to less than 94, the color characteristics of the monomer composition for synthesizing recycled plastics of the one embodiment deteriorate.

Meanwhile, when the color coordinate a* value of the monomer composition for synthesizing recycled plastics of the one embodiment excessively increases to more than 1.5, the monomer composition for synthesizing recycled plastics of the one embodiment represents a color excessively leaning toward red, resulting in deterioration of the color characteristics. Further, when the color coordinate a* value of the monomer composition for synthesizing recycled plastics of the one embodiment excessively decreases to less than 0.01, the monomer composition for synthesizing recycled plastics of the one embodiment represents a color excessively leaning toward green, resulting in deterioration of the color characteristics.

Examples of the method for measuring the color coordinates L*, a*, b* values of the monomer composition for synthesizing recycled plastics of the one embodiment are not particularly limited, and various color characteristic measurement methods in the field of plastics can be applied without limitation.

However, the color coordinates L*, a*, and b* values of the monomer composition for synthesizing recycled plastics of the one embodiment can be measured in reflection mode using HunterLab UltraScan PRO Spectrophotometer as an example.

Meanwhile, the monomer composition for synthesizing recycled plastics of the one embodiment may have an aromatic diol compound yield whose lower limit range is 90% or more, or 92% or more, or 93% or more, or 94% or more, or 95% or more, or 96% or more, and whose upper limit range is 100% or less, or 98% or less, or 97% or less, or 96.4% or less. A numerical range from the lower limit to the upper limit can also be satisfied by combining the upper limit range and the lower limit range. The reason why the yield of the aromatic diol compound is increased to 90% or more is considered to be due to the method for preparing a monomer composition for synthesizing recycled plastics, which will be described later.

Examples of the method for measuring the yield of the aromatic diol compound of the monomer composition for synthesizing recycled plastics of the one embodiment are not particularly limited, and for example, the yield can be calculated according to the following Equation 2.

$$[\text{Equation 2}]$$

$$\text{Yield } (\%) = W_1 \,/\, W_0$$

wherein, in Equation 2, $W_0$ is the mass of the aromatic diol compound obtained at the time of 100% decomposition, and $W_1$ is the mass of the actually obtained aromatic diol compound.

In Equation 2, the mass of the aromatic diol compound can be measured by various commonly known mass measurement methods without limitation, and a balance can be used as an example.

In this manner, in the monomer composition for synthesizing recycled plastics of the one embodiment, the yield of the aromatic diol compound, which is the main object material to be recovered, is greatly increased to 90% or more, so that the efficiency of the recycling process for the polycarbonate-based resin can be improved.

The monomer composition for synthesizing recycled plastics of the one embodiment can be used as a raw material for preparing various recycled plastics (e.g., polycarbonate (PC)) which will be described later.

The monomer composition for synthesizing recycled plastics of the one embodiment may further include small amounts of other additives and solvents. Specific types of the additives or solvents are not particularly limited, and various materials widely used in the process of recovering the aromatic diol compound by a depolymerization of the polycarbonate-based resin can be applied without limitation.

The monomer composition for synthesizing recycled plastics of the one embodiment can be obtained by a method for preparing a monomer composition for synthesizing recycled plastics, which will be described later. That is, the monomer composition for synthesizing recycled plastics of the one embodiment corresponds to the result obtained through various processes of filtration, purification, washing, and drying in order to secure only the aromatic diol compound, which is the main object material to be recovered, with high purity after the depolymerization reaction of the polycarbonate-based resin.

## 2. Method for preparing a monomer composition for synthesizing recycled plastic

According to another embodiment of the invention, there can be provided a method for preparing the monomer composition for synthesizing recycled plastic, the method comprising the steps of: adding polycarbonate to an organic solvent to prepare a mixed solution; adding a glycol-based compound and an inorganic base catalyst to the mixed solution and stirring them; and obtaining an aromatic diol compound formed in the stirring step.

Specifically, according to the present invention, a polycarbonate can be decomposed to a glycol-based compound under mild conditions to thereby stably obtain bisphenol A, which is a high-purity monomer.

The polycarbonate is meant to include both a homopolymer and a copolymer containing a polycarbonate, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to contain two or more carbonates, a copolymer can be synthesized. The homopolymer or copolymer can include all of low-molecular compounds, oligomers, and polymers depending on the molecular weight range.

The polycarbonate-based resin can be applied regardless of various forms and types, such as a novel polycarbonate produced through synthesis, a recycled polycarbonate produced through a regeneration process, or polycarbonate waste.

In the present invention, the glycol-based compound may include both a glycol compound and a derivative compound thereof, and specifically, the glycol-based compound may include alkylene glycol. Specific examples of the alkylene glycol are not particularly limited, and conventionally known various alkylene glycols can be used without limitation. One example is ethylene glycol or propylene glycol.

Meanwhile, the organic solvent may include one or more solvents selected from the group consisting of tetrahydrofuran, toluene, methylene chloride, chloroform, dimethyl carbonate, ethylmethyl carbonate, diethyl carbonate and dipropyl carbonate. That is, the organic solvent may include tetrahydrofuran, toluene, methylene chloride, chloroform, dimethyl carbonate, ethylmethyl carbonate, diethyl carbonate, dipropyl carbonate, or a mixture of two or more thereof.

Specifically, when methylene chloride is used as the organic solvent, there is an advantage that the dissolution characteristics in polycarbonate can be improved and the reactivity can be improved.

The weight ratio of the glycol-based compound, organic solvent, and polycarbonate is not particularly limited, but as an example, the weight ratio between the glycol-based compound and polycarbonate may be 10 : 1 to 1 : 10, or 1 : 1 to 1 : 10, or 1 : 1 to 1 : 5, or 1 : 1 to 1 : 2, or 1 : 1.1 to 1 : 2, or 1 : 1.2 to 1 : 2.

Further, the weight ratio between the organic solvent and polycarbonate may be 10 : 1 to 1 : 10, or 10 : 1 to 1 : 1, or 10 : 1 to 2 : 1, or 5 : 1 to 2 : 1, or 3 : 1 to 2 : 1, or 3 : 1 to 2.5 : 1, or 10 : 1 to 5 : 1, or 7 : 1 to 5 : 1, or 7 : 1 to 6 : 1.

In addition, the weight ratio between the organic solvent and the glycol-based compound may be 20 : 1 to 1 : 20, or 10 : 1 to 1 : 10, or 1 : 1 to 10 : 1, or 2 : 1 to 10 : 1, or 2 : 1 to 5 : 1, or 3 : 1 to 4 : 1, or 10 : 1 to 20 : 1, or 10:1 to 15 : 1, or 11 : 1 to 13:1, or 12 : 1 to 13 : 1.

Specifically, by mixing the polycarbonate compound and an organic solvent within the above range, there is an advantage that the depolymerization reaction of the polymer can proceed at a desired level.

The inorganic base catalyst may be added in an amount of 0.1 part by weight to 10 parts by weight, or 0.1 part by weight to 5 parts by weight, or 0.1 part by weight to 1 part by weight, based on 100 parts by weight of polycarbonate. Specifically, by containing the inorganic base catalyst in the above content range, there is an advantage that economical catalytic reactions can proceed.

The inorganic base catalyst means a nonionic inorganic compound having basicity, and specifically, it may be sodium hydroxide (NaOH) or potassium hydroxide (KOH), preferably sodium hydroxide (NaOH), without being limited thereto. The use of the inorganic base catalyst allows the decomposition reaction to proceed under mild conditions, which is economically advantageous.

Meanwhile, the adding the glycol-based compound and the inorganic base catalyst to the mixed solution and stirring them may be stirring at 20 °C to 100 °C, or 50 °C to 100 °C, or 60 °C to 100 °C, or 70 °C to 100 °C, or 70 °C to 90 °C for 1 hour to 24 hours, or 1 hour to 12 hours, or 1 hour to 8 hours. The adding the glycol-based compound and the inorganic base catalyst to the mixed solution and stirring them may be stirring at 20°C to 100°C for 1 hour to 30 hours.

Specifically, the conditions are mild process conditions relative to the conventional pressurizing/high temperature process, and by performing stirring under the above conditions, the process can be performed in a mild process as compared to the pressurizing/high temperature process. In particular, when stirring at 70°C to 90°C for 1 to 12 hours, there is an advantage of obtaining the most efficient results in terms of reproducibility and acceptability.

Meanwhile, the step of obtaining the aromatic diol compound formed in the stirring step may comprise injecting the product obtained in the stirring step into a crystallization solvent to form aromatic diol compound crystals; and filtering the aromatic diol compound crystals. Specific equipment and conditions for adding the crystallization solvent and forming aromatic diol compound crystals are not limited, and various recrystallization processes that have been widely used in the technical field of recovering conventional aromatic diol compounds (bisphenol A) can be applied without limitation.

As an example of the crystallization solvent, water may be used.

Further, in the step of filtering the aromatic diol compound crystals, specific filtration equipment and conditions are not limited, and conventionally, various filtration processes that have been widely used in the technical field of recovering aromatic diol compounds (bisphenol A) can be applied without limitation. As an example of the filtration, filtration under reduced pressure can be used.

### 3. Recycled Plastic

According to another embodiment of the invention, a recycled plastic comprising a reaction product of the monomer composition for synthesizing recycled plastic of the one embodiment and a comonomer can be provided.

The details of the monomer composition for synthesizing recycled plastic of the one embodiment include all the contents described above in the one embodiment and the other embodiment.

Examples corresponding to the recycled plastic are not particularly limited, and various plastics synthesized from aromatic diol compounds such as bisphenol A, and carbonate precursors such as dimethyl carbonate, diethyl carbonate, or ethylmethyl carbonate as a monomer can be applied without limitation, and a more specific example may be a polycarbonate-based resin.

The polycarbonate-based resin is meant to include both a homopolymer and a copolymer containing a polycarbonate repeating unit, and collectively refers to a reaction product obtained through a polymerization reaction or a copolymerization reaction of a monomer containing an aromatic diol compound and a carbonate precursor. When it contains one carbonate repeating unit obtained by using only one aromatic diol compound and one carbonate precursor, a homopolymer can be synthesized. In addition, when one aromatic diol compound and two or more carbonate precursors are used as the monomer, or two or more aromatic diol compounds and one carbonate precursor are used, or one or more other diols is used in addition to the one aromatic diol compound and the one carbonate precursor to thereby contain two or more carbonates, a copolymer can be synthesized. The homopolymer or copolymer can include all of low-molecular compounds, oligomers, and polymers depending on the molecular weight range.

More specifically, in the recycled plastic containing the reaction product of the monomer composition for synthesizing the recycled plastic of the one embodiment and the comonomer, a carbonate precursor can be used as the comonomer. Specific examples of the carbonate precursor include phosgene, triphosgene, diphosgene, bromophosgene, dimethyl carbonate, diethyl carbonate, dibutyl carbonate, dicyclohexyl carbonate, diphenyl carbonate, ditolyl carbonate, bis(chlorophenyl)carbonate, m-cresyl carbonate, dinaphthyl carbonate, bis(diphenyl) carbonate or bishaloformate.

Examples of the reaction process of the monomer composition for synthesizing recycled plastic and a comonomer for synthesizing the polycarbonate-based resin are not particularly limited, and various well-known methods for preparing polycarbonate can be applied without limitation.

However, in one example of the polycarbonate preparation method, a polycarbonate preparation method including the step of polymerizing a composition containing a monomer composition for synthesizing recycled plastic and a comonomer can be used. At this time, the polymerization can be carried out by interfacial polymerization, and during interfacial polymerization, polymerization reaction is possible at normal pressure and low temperature, and the molecular weight is easy to control.

The polymerization temperature may be 0°C to 40°C, and the reaction time may be 10 minutes to 5 hours. In addition, the pH during the reaction may be maintained at 9 or more or 11 or more.

The solvent that can be used for the polymerization is not particularly limited as long as it is a solvent used for polymerization of polycarbonate in the art, and as an example, halogenated hydrocarbons such as methylene chloride and chlorobenzene can be used.

Moreover, the polymerization can be carried out in the presence of an acid binder. As the acid binder, an alkali metal hydroxide such as sodium hydroxide or potassium hydroxide, or an amine compound such as pyridine can be used.

Further, in order to adjust the molecular weight of the polycarbonate during the polymerization, polymerization can be performed in the presence of a molecular weight modifier. An alkylphenol having 1 to 20 carbon atoms may be used as the molecular weight modifier, and specific examples thereof include p-tert-butylphenol, p-cumylphenol, decylphenol, dodecylphenol, tetradecylphenol, hexadecylphenol, octadecylphenol, eicosylphenol, docosylphenol or triacontylphenol. The molecular weight modifier can be added before, during or after the initiation of polymerization. The molecular weight modifier may be used in an amount of 0.01 to 10 parts by weight, or 0.1 to 6 parts by weight, based on 100 parts by weight of the aromatic diol compound, and a desired molecular weight can be obtained within this range.

In addition, in order to promote the polymerization reaction, a reaction accelerator such as a tertiary amine compound, a quaternary ammonium compound, or a quaternary phosphonium compound, including triethylamine, tetra-n-butylammonium bromide, or tetra-n-butylphosphonium bromide can be further used.

**4. Molded Product**

According to yet another embodiment of the invention, a molded article comprising the recycled plastic of the other embodiment can be provided. The details of the recycled plastic includes all the contents described above in the other embodiments.

The molded article can be obtained by applying the recycled plastic to various known plastic molding methods without limitation. As an example of the molding method, injection molding, foam injection molding, blow molding, or extrusion molding may be mentioned.

Examples of the molded article are not particularly limited, and can be applied to various molded articles using plastic without limitation. Examples of the molded article include automobiles, electrical and electronic products, communication products, daily necessities, building materials, optical components, exterior materials, and the like.

The molded article may further include one or more additives selected from the group consisting of an antioxidant, a plasticizer, an antistatic agent, a nucleating agent, a flame retardant, a lubricant, an impact enhancer, an optical brightener, an ultraviolet absorber, a pigment and a dye, if necessary, in addition to the recycled plastic of the other embodiments,

An example of the manufacturing method of the molded article may include a step of mixing the recycled plastic of the other embodiment and an additive well using a mixer, extrusion-molding the mixture with an extruder to produce pellets, drying the pellets, and then injecting them with an injection molding machine.

**[Advantageous Effects]**

According to the present invention, a monomer composition for synthesizing recycled plastic that contains an aromatic diol compound having high purity and excellent optical properties recovered through recycling by chemical decomposition of a polycarbonate-based resin, a method for preparing the same, and a recycled plastic and molded product using the same can be provided.

**[DETAILED DESCRIPTION OF THE EMBODIMENTS]**

The present invention will be explained in more detail with reference to the following examples. However, these examples are for illustrative purposes only, and the scope of the present invention is not limited thereto.

**<EXAMPLE: Preparation of recycled bisphenol A monomer composition>**

Example 1

80 g of Methylene chloride and 30 g of polycarbonate were added to a 250 ml three-neck flask, and then stirred.
Then, 22 g of ethylene glycol and 0.05 g of sodium hydroxide (NaOH) as a catalyst were added thereto, and stirred at 70°C for 24 hours. (Mass ratio of polycarbonate: methylene chloride: ethylene glycol: sodium hydroxide = 30: 80: 22: 0.05).
When the reaction was completed, the reaction product was added to water, and the crystallized bisphenol A was filtered under reduced pressure to obtain bisphenol A, thereby preparing a recycled bisphenol A monomer composition.

Example 2

A recycled bisphenol A monomer composition was prepared in the same manner as in Example 1, except that in Example 1, the mass ratio of polycarbonate: methylene chloride: ethylene glycol: sodium hydroxide was changed to 30: 200: 16: 0.16.

Example 3

80 g of Methylene chloride and 30 g of polycarbonate were added to a 250 ml three-neck flask, and then stirred.
Then, 27 g of propylene glycol and 0.05 g of sodium hydroxide (NaOH) as a catalyst were added thereto, and stirred at 70°C for 24 hours. (Mass ratio of polycarbonate: methylene chloride: propylene glycol: sodium hydroxide = 30: 80: 27: 0.05).
When the reaction was completed, the reaction product was added to water, and the crystallized bisphenol A was filtered under reduced pressure to obtain bisphenol A, thereby preparing a recycled bisphenol A monomer composition.

Example 4

A recycled bisphenol A monomer composition was prepared in the same manner as in Example 1, except that in Example 1, the mass ratio of polycarbonate: methylene chloride: ethylene glycol: sodium hydroxide was changed to 30: 200: 20: 0.16.

**<COMPARATIVE EXAMPLE: Preparation of recycled bisphenol A monomer composition>**

Comparative Example 1

20 g of Polycarbonate was added to a 250 ml three-neck flask, and then stirred.
Then, 22 g of ethylene glycol and 0.05 g of sodium hydroxide (NaOH) as a catalyst were added thereto, and stirred at 130°C for 24 hours.
When the reaction was completed, the reaction product was added to water, and the crystallized bisphenol A was filtered under reduced pressure to obtain bisphenol A, thereby preparing a recycled bisphenol A monomer composition.

Comparative Example 2

60 ml of Methylene chloride, 30 ml of methanol and 0.6 g of sodium hydroxide (NaOH) were added to a 250 ml three-neck flask, and then stirred.
Then, 30 g of polycarbonate was added thereto, and stirred at 40°C for 5 hours.
When the reaction was completed, the reaction product was added to water, and the crystallized bisphenol A was filtered under reduced pressure to obtain bisphenol A, thereby preparing a recycled bisphenol A monomer composition.

Comparative Example 3

60 ml of Toluene, 30 ml of methanol and 0.6 g of sodium hydroxide (NaOH) were added to a 250 ml three-neck flask, and then stirred.
Then, 30 g of polycarbonate was added thereto, and stirred at 60°C for 5 hours.
When the reaction was completed, the reaction product was added to water, and the crystallized bisphenol A was filtered under reduced pressure to obtain bisphenol A, thereby preparing a recycled bisphenol A monomer composition.

**<Experimental Example>**

The physical properties of the recycled bisphenol A monomer composition or by-products obtained in Examples and Comparative Examples were measured by the following methods, and the results are shown in Table 1 below.

**1. Color Coordinates (L\*, a\*, and b\*)**

The color coordinates of the recycled bisphenol A monomer composition were analyzed in reflection mode using HunterLab UltraScan PRO Spectrophotometer.

**2. Ratio of BPA Derivative (MHE-BPA, BHE-BPA) Impurity**

1 wt% of Recycled bisphenol A monomer composition was dissolved in acetonitrile (ACN) solvent under the conditions of normal pressure and 20 to 30°C, and then the purity of bisphenol A (BPA) was analyzed by a Waters HPLC system (e2695 separation module, 2998 PDA detector) using a UG 120 (4.6 mm I.DX 50 mm).
The peak area ratio (unit: %) of BPA derivative (monohydroxyethyl-bisphenol A [MHE-BPA] and bishydroxyethyl-bisphenol A [BHE-BPA]) impurities relative to 100% of the total HPLC peak area was measured according to the following Equation 1:

[Equation 1]

Ratio of aromatic diol compound derivative impurity (%) = (Peak area of the aromatic diol compound derivative in HPLC / Total peak area in HPLC) X 100.

In Chemical Formula 1, the peak of bisphenol A derivative in HPLC are specifically as follows, and in Chemical Formula 1, the peak area of the bisphenol A derivative in HPLC is the total peak area of MHE-BPA and BHE-BPA.

Monohydroxyethyl-bisphenol A [MHE-BPA]: peak at retention time of 2.84 min

Bishydroxyethyl-bisphenol A [BHE-BPA]: peak at retention time of 2.61 min

Further, the HPLC measurement conditions are as follows.

<HPLC conditions>

① Column: UG 120 (4.6mm I.DX 50mm)

(2) Column Temp: 40°C

(3) Injection volume: $10\mu\ell$

④ Flow: THF:ACN:water=15:15:70, volume=1.23ml/min (total=10 min)

(5) Detector: 245nm

**3. BPA Yield**

The weight of BPA produced when the polycarbonate used in the reaction was 100% decomposed was measured, and the weight of the obtained BPA was measured, so that the yield of BPA was calculated as shown in the following Equation 2.

[Equation 2]

$$Yield (\%) = W_1 / W_0$$

wherein, in Equation 2, $W_0$ is the mass of the aromatic diol compound obtained at the time of 100% decomposition, and $W_1$ is the mass of the actually obtained aromatic diol compound. Specifically, when about 100 g of polycarbonate is decomposed, the mass of BPA obtained at the time of 100% decomposition in theory is 89 g. If the mass of the actually obtained BPA is 80 g, the yield is 80/89 * 100% = 90%.

[Table 1]

| Measurement results of Experimental Examples | | | | | |
|---|---|---|---|---|---|
| Category | L* | a* | b* | Ratio of BPA derivative impurity (%) | BPA yield (%) |
| Example 1 | 96.554 | 0.20 | 0.77 | 0.10 | 93.2 |
| Example 2 | 97.207 | 0.10 | 0.58 | 0.05 | 96.4 |
| Example 3 | 96.874 | 0.23 | 0.81 | 0.12 | 94.1 |
| Example 4 | 96.904 | 0.23 | 1.02 | 0.43 | 95.4 |
| Comparative Example 1 | - | - | - | 22.9 | - |
| Comparative Example 2 | 91.95 | 2.667 | 3.847 | - | 85 |
| Comparative Example 3 | 91.73 | 2.554 | 3.457 | - | 86 |

As shown in Table 1, the recycled bisphenol A monomer compositions obtained in Examples 1 to 4 exhibited color coordinates L* of 96.554 to 97.207, a* of 0.10 to 0.23, and b* of 0.58 to 1.02, showing excellent optical properties. Also, for the recycled bisphenol A monomer compositions obtained in Examples 1 to 4, the ratio of BPA derivative impurity was measured as low as 0.05% to 0.43%. In addition, for the recycled bisphenol A monomer compositions obtained in Examples 1 to 4, the yield of bisphenol A was measured as high as 93.2% to 96.4%.

On the other hand, for the recycled bisphenol A monomer composition obtained in Comparative Example 1, the ratio of BPA derivative impurity was measured to be 22.9%, which was higher than that of Examples.

In addition, the recycled bisphenol A monomer compositions obtained in Comparative Examples 2 and 3 had color coordinates L* of 91.73 to 91.95, a* of 2.554 to 2.667, and b* of 3.457 to 3.847, showing poor optical properties compared to Examples, and the yield of bisphenol A was 85% to 86%, which was lower than that of Examples.

**Claims**

1. A monomer composition for synthesizing recycled plastic,

   which comprises an aromatic diol compound,
   wherein a ratio of aromatic diol compound derivative impurity according to the following Equation 1 is 10% or less,
   wherein the monomer composition has a color coordinate b* of 0.01 to 2, and
   wherein the monomer composition is recovered from a polycarbonate-based resin:

   [Equation 1]

   Ratio of aromatic diol compound derivative impurity= (Peak area of the aromatic diol compound derivative in HPLC / Total peak area in HPLC) X 100%.

2. The monomer composition for synthesizing recycled plastic according to claim 1 wherein:
   in Equation 1, the peak area of the aromatic diol compound derivative in HPLC is the total peak area of each of one or more aromatic diol compound derivatives.

3. The monomer composition for synthesizing recycled plastic according to claim 1 wherein:
   the derivative of the aromatic diol compound comprises one or more compounds selected from the group consisting of monohydroxyethyl-bisphenol A and bishydroxyethyl-bisphenol A.

4. The monomer composition for synthesizing recycled plastic according to claim 1 wherein:
   the monomer composition for synthesizing recycled plastic has a color coordinate L* of 94 to 99.

5. The monomer composition for synthesizing recycled plastic according to claim 1 wherein:
   the monomer composition for synthesizing recycled plastic has a color coordinate a* of 0.01 to 1.5.

6. The monomer composition for synthesizing recycled plastic according to claim 1 wherein:
   the aromatic diol compound is recovered from the polycarbonate-based resin used to recover the monomer composition for synthesizing recycled plastic.

7. A method for preparing the monomer composition for synthesizing recycled plastic of claim 1, the method comprising the steps of:

   adding polycarbonate to an organic solvent to prepare a mixed solution;
   adding a glycol-based compound and an inorganic base catalyst to the mixed solution and stirring them; and
   obtaining an aromatic diol compound formed in the stirring step.

8. The method for preparing a monomer composition for synthesizing recycled plastic according to claim 7, wherein:
   the glycol-based compound comprises alkylene glycol.

9. The method for preparing a monomer composition for synthesizing recycled plastic according to claim 7, wherein:
   the organic solvent comprises one or more solvents selected from the group consisting of tetrahydrofuran, toluene, methylene chloride, chloroform, dimethyl carbonate, ethylmethyl carbonate, diethyl carbonate and dipropylcarbonate.

10. The method for preparing a monomer composition for synthesizing recycled plastic according to claim 7, wherein:

a weight ratio between the organic solvent and the glycol-based compound is 20:1 to 1:20.

11. The method for preparing a monomer composition for synthesizing recycled plastic according to claim 7, wherein: the inorganic base catalyst is added in an amount of 0.1 to 10 parts by weight based on 100 parts by weight of polycarbonate.

12. The method for preparing a monomer composition for synthesizing recycled plastic according to claim 7, wherein: the inorganic base catalyst comprises sodium hydroxide (NaOH) or potassium hydroxide (KOH).

13. The method for preparing a monomer composition for synthesizing recycled plastic according to claim 7, wherein: the adding a glycol-based compound and an inorganic base catalyst to the mixed solution and stirring them is stirring at 20°C to 100°C for 1 hour to 30 hours.

14. The method for preparing a monomer composition for synthesizing recycled plastic according to claim 7, wherein:

the obtaining the aromatic diol compound formed in the stirring step comprises,
injecting the product obtained in the stirring step into a crystallization solvent to form aromatic diol compound crystals; and
filtering the aromatic diol compound crystals.

15. A recycled plastic comprising a reaction product of the monomer composition for synthesizing recycled plastic of claim 1 and a comonomer.

16. A molded product comprising the recycled plastic of claim 15.

## INTERNATIONAL SEARCH REPORT

| International application No. |
|---|
| **PCT/KR2023/004933** |

**A.     CLASSIFICATION OF SUBJECT MATTER**

**C08J 11/18**(2006.01)i; **C08J 11/28**(2006.01)i; **C08J 11/16**(2006.01)i; **C07C 37/00**(2006.01)i; **C07C 39/16**(2006.01)i; **C07D 317/38**(2006.01)i

According to International Patent Classification (IPC) or to both national classification and IPC

**B.     FIELDS SEARCHED**

Minimum documentation searched (classification system followed by classification symbols)

C08J 11/18(2006.01); B01J 31/02(2006.01); C07C 37/52(2006.01); C07C 37/66(2006.01); C07C 37/72(2006.01); C08G 64/24(2006.01); C08K 7/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 재활용 플라스틱(recycled plastics), 폴리카보네이트(polycarbonate), 방향족 디올 (aromatic diol), 무기염기 촉매(inorganic base catalyst)

**C.     DOCUMENTS CONSIDERED TO BE RELEVANT**

| Category* | Citation of document, with indication, where appropriate, of the relevant passages | Relevant to claim No. |
|---|---|---|
| Y | QUARANTA, E. et al. Chemical Recycling of Poly(bisphenol A carbonate) by Glycolysis under 1,8-Diazabicyclo[5.4.0]undec-7-ene Catalysis. ACS Omega. 2018, vol. 3, pp. 7261-7268.<br>See page 7263. | 1-16 |
| Y | 허미선 등. 폴리카보네이트 해중합을 이용한 Bisphenol A계 Bishydroxyethyl Ether 화합물 생성 특성. Korean Chemical Engineering Research. 2010, vol. 48, no. 2, pp. 164-171 (HEO, Miseon et al. Synthesis of Bishydroxyethyl Ether of Bisphenol A(BHE-BPA) Through the Depolymerization of Polycarbonate.)<br>See page 165. | 1-16 |
| Y | KR 10-2021-0082708 A (KTL CO., LTD.) 06 July 2021 (2021-07-06)<br>See claims 8-9. | 15-16 |
| A | KR 10-2008-0008333 A (TEIJIN CHEMICALS, LTD.) 23 January 2008 (2008-01-23)<br>See entire document. | 1-16 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 July 2023** | **18 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 345 127 A1**

**INTERNATIONAL SEARCH REPORT**

| International application No. |
| --- |
| **PCT/KR2023/004933** |

**C. DOCUMENTS CONSIDERED TO BE RELEVANT**

| Category* | Citation of document, with indication, where appropriate, of the relevant passages | Relevant to claim No. |
| --- | --- | --- |
| A | KR 10-2022-0023451 A (LG CHEM, LTD.) 02 March 2022 (2022-03-02)<br>See entire document. | 1-16 |

Form PCT/ISA/210 (second sheet) (July 2022)

**INTERNATIONAL SEARCH REPORT**
Information on patent family members

International application No.

**PCT/KR2023/004933**

| Patent document cited in search report | | | Publication date (day/month/year) | Patent family member(s) | | | Publication date (day/month/year) |
|---|---|---|---|---|---|---|---|
| KR | 10-2021-0082708 | A | 06 July 2021 | None | | | |
| KR | 10-2008-0008333 | A | 23 January 2008 | CN | 101166705 | A | 23 April 2008 |
| | | | | EP | 1873135 | A1 | 02 January 2008 |
| | | | | EP | 1873135 | A4 | 04 June 2008 |
| | | | | JP | 2008-114893 | A1 | 11 December 2008 |
| | | | | JP | 4679577 | B2 | 27 April 2011 |
| | | | | KR | 10-1166005 | B1 | 18 July 2012 |
| | | | | US | 2009-0170969 | A1 | 02 July 2009 |
| | | | | US | 7750057 | B2 | 06 July 2010 |
| | | | | WO | 2006-114893 | A1 | 02 November 2006 |
| KR | 10-2022-0023451 | A | 02 March 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220045959
- KR 1020230044308